# EUROPEAN PATENT APPLICATION

(11) **EP 2 879 068 A1**
(43) Date of publication of application: **03.06.2015**
(21) Application number: 14193970.2
(22) Date of filing: 20.11.2014
(51) Int. Cl.: G06F 19/00

(54) **Biological information measuring apparatus, biological information measuring system, individual registration information registering method, and program**

(30) Priority: 28.11.2013 JP 2013246547
(71) Applicant: Tanita Corporation, Tokyo (JP)
(72) Inventor: Suzuki, Shun, Itabashi-ku, Tokyo (JP); Fukada, Kosei, Itabashi-ku, Tokyo (JP)
(74) Representative: Grünecker Patent- und Rechtsanwälte PartG mbB

(57) **Abstract**

A biological information measuring apparatus includes: an individual information storage unit configured to store individual information for a plurality of persons in which individual registration information and biological information are associated; a transmission request reception unit configured to receive a transmission request of the individual registration information; an individual registration information transmission unit configured to transmit the individual registration information for a plurality of persons stored in the individual information storage unit based on the transmission request; a registration request reception unit configured to receive a registration request in which designation of any of the individual registration information for a plurality of persons and a registration content are associated; and a registration unit configured to register the registration content on the individual registration information designated in the registration request of the individual registration information stored in the individual information storage unit.

## Description

### BACKGROUND

### Field of the Invention

The present invention relates to a biological information measuring apparatus, a biological information measuring system, an individual registration information registering method, and a program.

### Background

A biological information measuring apparatus that performs communication with a terminal apparatus has been proposed.

For example, a biological information measuring apparatus disclosed in Japanese Patent Application, Publication No. 2009-240530A is a biological information measuring apparatus which performs mutual authentication between the biological information measuring apparatus and an external terminal to form a communication link of a bidirectional communication and transmits and receives data via the communication link. In the biological information measuring apparatus, a communication link of a bidirectional communication is formed, and then the communication link is temporarily released during a measurement operation. When the measurement operation is finished, the temporarily released communication link is formed again.

In addition, Japanese Patent Application, Publication No. 2009-240530A discloses that the bidirectional communication makes it possible to transmit physical information relating to a user such as, for example, age, body height, and body weight, from an external terminal toward the biological information measuring apparatus, enables multi-functionalized measurement of biological information, and also makes it easy to manage measurement data of the user.

### SUMMARY

When the biological information measuring apparatus stores information relating to a user received from the terminal apparatus, there is a possibility that information which is already stored in the biological information measuring apparatus is overwritten by mistake with new information, and the information which has been stored in the biological information measuring apparatus is lost.

An object of an aspect of the present invention is to provide a biological information measuring apparatus, a biological information measuring system, an individual registration information registering method, and a program capable of reducing a possibility that information relating to a user which is already stored in the biological information measuring apparatus is overwritten by mistake.

A biological information measuring apparatus according to an aspect of the present invention includes: an individual information storage unit configured to store individual information for a plurality of persons in which individual registration information and biological information are associated; a transmission request reception unit configured to receive a transmission request of the individual registration information; an individual registration information transmission unit configured to transmit the individual registration information for a plurality of persons stored in the individual information storage unit based on the transmission request; a registration request reception unit configured to receive a registration request in which designation of any of the individual registration information for a plurality of persons and a registration content are associated; and a registration unit configured to register the registration content on the individual registration information designated in the registration request of the individual registration information stored in the individual information storage unit.

The individual information storage unit may store the individual registration information including update timing information, the registration request reception unit may receive the registration request including update timing information in the individual registration information, and the registration unit may compare the update timing information stored in the individual information storage unit with the update timing information included in the registration request and may perform registration of individual registration information when the registration unit determines that an update timing indicated by the update timing information included in the registration request is newer.

The registration unit may request, to a transmission side of the registration request, the update of the individual registration information when the registration unit determines that an update timing indicated by the update timing information stored in the individual information storage unit is newer than an update timing indicated by the update timing information included in the registration request.

The biological information measuring apparatus may include: a registration presence or absence determination unit configured to determine whether or not the individual registration information designated in the registration request is registered as the individual registration information stored in the individual information storage unit; an overwrite approval or rejection confirmation request transmission unit configured to transmit a confirmation request of overwrite approval or rejection when the registration presence or absence determination unit determines that registration is present; and an overwrite approval or rejection reply reception unit configured to receive a reply to the confirmation request of overwrite approval or rejection, wherein the registration unit may prevent registration of information indicated in the reply as overwrite rejection.

The biological information measuring apparatus may include a color classification display unit configured to perform display of a color when biological information is measured, wherein the individual information storage unit may store the individual registration information including color information, and the color classification display unit may display a color associated with a biological information measurement object person in the individual registration information.

The registration request reception unit may receive the registration request including information indicating a notification object item of the individual registration information, the individual information storage unit may store the individual information including information indicating a notification object item of the individual registration information, and the individual registration information transmission unit may transmit the individual registration information for a plurality of persons including information indicating a notification object item of the individual registration information.

A biological information measuring system according to another aspect of the present invention includes: a biological information measuring apparatus; and a terminal apparatus, wherein the biological information measuring apparatus includes: an individual information storage unit configured to store individual information for a plurality of persons in which individual registration information and biological information are associated; a transmission request reception unit configured to receive a transmission request of the individual registration information from the terminal apparatus; an individual registration information transmission unit configured to transmit the individual registration information for a plurality of persons stored in the individual information storage unit to the terminal apparatus based on the transmission request; a registration request reception unit configured to receive a registration request in which designation of any of the individual registration information for a plurality of persons and a registration content are associated, from the terminal apparatus; and a registration unit configured to register the registration content on the individual registration information designated in the registration request of the individual registration information stored in the individual information storage unit, and the terminal apparatus includes: a transmission request transmission unit configured to transmit a transmission request of the individual registration information to the biological information measuring apparatus; an individual registration information reception unit configured to receive the individual registration information for a plurality of persons from the biological information measuring apparatus; an individual registration information notification unit configured to notify the individual registration information for a plurality of persons received by the individual registration information reception unit; an operation input unit configured to accept a selection operation of any of the individual registration information for a plurality of persons; and a registration request transmission unit configured to transmit a registration request in which designation of the individual registration information selected in the selection operation and a registration content on the individual registration information are associated, to the biological information measuring apparatus.

An individual registration information registering method according to still another aspect of the present invention is an individual registration information registering method of a biological information measuring apparatus including an individual information storage unit configured to store individual information for a plurality of persons in which individual registration information and biological information are associated, the method including: a transmission request reception step of receiving a transmission request of the individual registration information; an individual registration information transmission step of transmitting the individual registration information for a plurality of persons stored in the individual information storage unit based on the transmission request; a registration request reception step of receiving a registration request in which designation of any of the individual registration information for a plurality of persons and a registration content are associated; and a registration step of registering the registration content on the individual registration information designated in the registration request of the individual registration information stored in the individual information storage unit.

A program according to still another aspect of the present invention is a program for causing a terminal apparatus configured to perform communication with a biological information measuring apparatus to execute: a transmission request transmission step of transmitting a transmission request of individual registration information to the biological information measuring apparatus; an individual registration information reception step of receiving the individual registration information for a plurality of persons from the biological information measuring apparatus; an individual registration information notification step of notifying the individual registration information for a plurality of persons received in the individual registration information reception step; an operation input step of accepting a selection operation of any of the individual registration information for a plurality of persons; and a registration request transmission step of transmitting a registration request in which designation of the individual registration information selected in the selection operation and a registration content on the individual registration information are associated, to the biological information measuring apparatus.

According to an aspect of the present invention, it is able to reduce a possibility that information relating to a user which is already stored in the biological information measuring apparatus is overwritten by mistake.

### BRIEF DESCRIPTION OF THE DRAWINGS

FIG. 1 is a schematic block diagram showing a functional configuration of a biological information measuring system according to an embodiment of the present invention.
FIG. 2 is a diagram showing an example of a data structure of individual information stored in an individual information storage unit according to the embodiment.
FIG. 3 is a schematic outline diagram showing an example of an external form of a body fat scale according to the embodiment.
FIG. 4 is a schematic outline diagram showing an example of an external form of a smartphone according to the embodiment.
FIG. 5 is a diagram showing a display example of individual registration information stored in the individual information storage unit, by a display unit according to the embodiment.
FIG. 6 is a sequence diagram showing an example of a process sequence in which pairing between the body fat scale and the smartphone is performed according to the embodiment.
FIG. 7 is a sequence diagram showing an example of a process sequence in which the body fat scale measures biological information and the smartphone displays a measurement result according to the embodiment.
FIG. 8 is a diagram showing an example of a data structure of individual registration information stored in a storage unit, in an operation in which the smartphone transmits only part of the individual registration information according to the embodiment.
FIG. 9 is a diagram showing an example of a data structure of individual registration information stored in the individual information storage unit and the storage unit, in an operation in which whether the update of the individual registration information is new or old is determined according to the embodiment.
FIG. 10 is a diagram showing an example of a data structure of individual registration information stored in the individual information storage unit and the storage unit, in an operation in which color classification display of each user is performed according to the embodiment.
FIG. 11 is a schematic outline diagram showing an example of an external form of a body fat scale in an operation in which color classification display of each user is performed according to the embodiment.
FIG. 12 is a diagram showing a display example of a smartphone in an operation in which color classification display of each user is performed according to the embodiment.
FIG. 13 is a diagram showing an example of a data structure of individual registration information stored in the individual information storage unit and the storage unit, in an operation in which a user can select an item to be a display object, of the items of individual registration information according to the embodiment.
FIG. 14 is a diagram showing a display example of a smartphone in an operation in which a user can select an item to be a display object, of the items of individual registration information according to the embodiment.
FIG. 15 is a schematic block diagram showing a functional configuration of a biological information measuring system according to a modified example of the present embodiment.
FIG. 16 is a sequence diagram showing an example of a process sequence in which pairing between the body fat scale and the smartphone is performed according to the modified example.

### BRIEF DESCRIPTION OF THE REFERENCE NUMERALS

1,2 BIOLOGICAL INFORMATION MEASURING SYSTEM
10, 12 BODY FAT SCALE
20, 22 SMARTPHONE
110, 210 COMMUNICATION UNIT
120, 220 DISPLAY UNIT
130, 230 OPERATION INPUT UNIT
140, 240 STORAGE UNIT
141 INDIVIDUAL INFORMATION STORAGE UNIT
150 BIOLOGICAL INFORMATION SENSOR UNIT
180, 280 CONTROL UNIT
181, 281 COMMUNICATION CONTROL UNIT
182, 282 DISPLAY CONTROL UNIT
183, 283 INPUT PROCESS UNIT
184, 284 STORAGE PROCESS UNIT
185 BIOLOGICAL INFORMATION ACQUISITION UNIT
186 COMMUNICATION PROCESS UNIT
187, 286 REGISTRATION PRESENCE OR ABSENCE DETERMINATION UNIT
188, 287 OVERWRITE APPROVAL OR REJECTION CONFIRMATION PROCESS UNIT
285 APPLICATION IMPLEMENTATION UNIT

### DESCRIPTION OF THE EMBODIMENTS

Hereinafter, the present invention is described though embodiments of the invention. However, the following embodiments do not limit the invention of the claims. Furthermore, not all of the combinations of the characteristics described in the embodiments are essential to the problem-solving means of the invention.

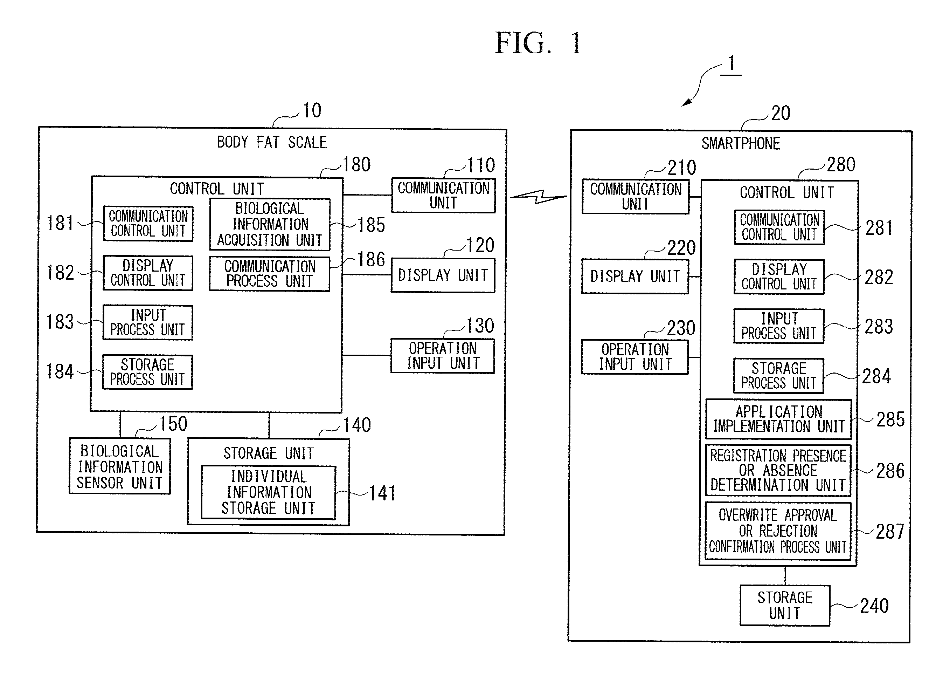

FIG. 1 is a schematic block diagram showing a functional configuration of a biological information measuring system according to an embodiment of the present invention. In the drawing, a biological information measuring system 1 is provided with a body fat scale 10 and a smartphone 20.

The body fat scale 10 is provided with a communication unit 110, a display unit 120, an operation input unit 130, a storage unit 140, a biological information sensor unit 150, and a control unit 180. The storage unit 140 is provided with an individual information storage unit 141. The control unit 180 is provided with a communication control unit 181, a display control unit 182, an input process unit 183, a storage process unit 184, a biological information acquisition unit 185, and a communication process unit 186. The smartphone 20 is provided with a communication unit 210, a display unit 220, an operation input unit 230, a storage unit 240, and a control unit 280. The control unit 280 is provided with a communication control unit 281, a display control unit 282, an input process unit 283, a storage process unit 284, an application implementation unit 285, a registration presence or absence determination unit 286, and an overwrite approval or rejection confirmation process unit 287.

The body fat scale 10 measures the body weight and body fat percentage of a user and stores a history of measurement results.

The body fat scale 10 is capable of registering a plurality of users. The body fat scale 10 stores a history of measurement results for each user. Further, the body fat scale 10 performs communication with the smartphone 20 and exchanges a variety of information relating to the user of the body fat scale 10, such as a measurement result of biological information.

The body fat scale 10 corresponds to an example of a biological information measuring apparatus. The biological information measuring apparatus of the present embodiment is not limited to a body fat scale, and can be various kinds of biological information measuring apparatuses such as a blood pressure monitor or an electrocardiogram monitor.

The storage unit 140 is configured to include a storage device provided in the body fat scale 10 and stores a variety of information in accordance with control of the storage process unit 184.

The individual information storage unit 141 stores individual information in which individual registration information and biological information are associated, for a plurality of persons.

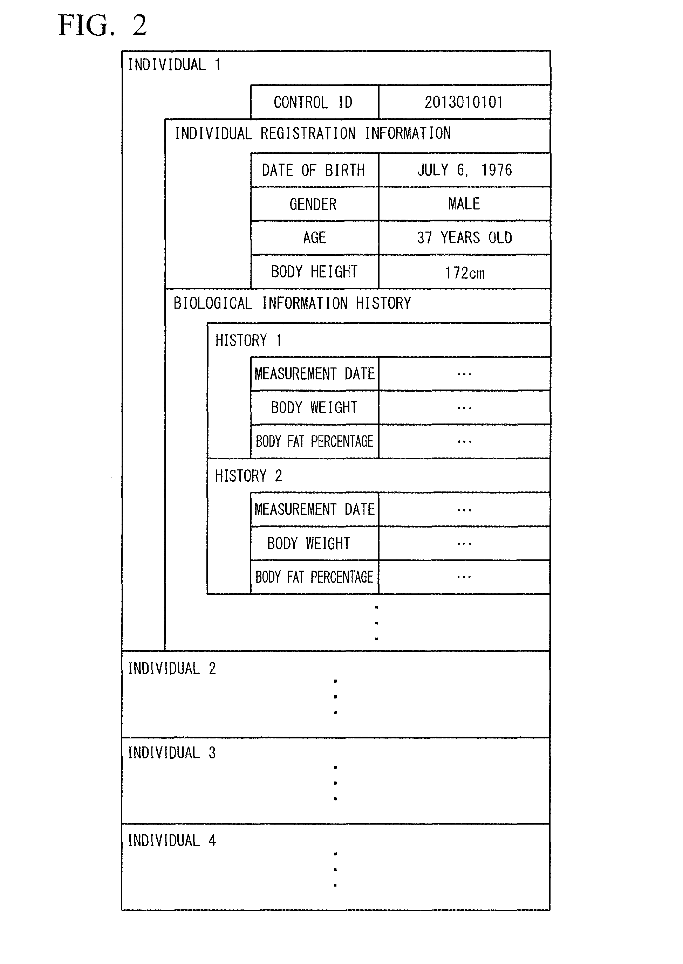

FIG. 2 is a diagram showing an example of a data structure of individual information stored in the individual information storage unit 141. The term "individual information" here denotes information which the body fat scale 10 stores for each user.

In the example of FIG. 2, the individual information storage unit 141 stores individual information for four persons of Individual 1 to Individual 4. The individual information each includes a control ID (Identifier, identification information), individual registration information, and a biological information history.

The control ID is information for identifying the individual information for each person. For example, the storage process unit 184 sets the control ID at the time of registration of the individual information.

The individual registration information is information, such as gender or body height of the user, which is preliminarily registered in individual information storage unit 141 for each user before measurement of the biological information. In the example of FIG. 2, the individual information storage unit 141 stores, as the individual registration information, the date of birth, gender, age, and body height of the user. For example, the smartphone 20 accepts an input of information of the date of birth, gender, and body height by a user operation and transmits the information to the body fat scale 10, and the storage process unit 184 causes the individual information storage unit 141 to store the information. In addition, the storage process unit 184 calculates the age from the date of birth and current date and causes the individual information storage unit 141 to store the age.

The biological information history is a history of biological information measured by the body fat scale 10. In the example of FIG. 2, the individual information storage unit 141 stores, as the biological information history, the measurement date, body weight, and body fat percentage, for each time of measurement.

The individual information which the individual information storage unit 141 stores may include unregistered information. For example, the individual information of Individual 1 may be registered, and the individual information of Individual 2 may be unregistered. The unregistered information has a data value (for example, null string) capable of identifying that the information is unregistered.

With reference back to FIG. 1, the communication unit 110 performs communication with the communication unit 210 of the smartphone 20 in accordance with control of the communication control unit 181 and exchanges a variety of information. The communication unit 110 is configured to include a communication circuit provided in the body fat scale 10.

Specifically, the communication unit 110 receives, from the communication unit 210 of the smartphone 20, a transmission request of the individual registration information stored in the individual information storage unit 141 when individual registration information is registered from the smartphone 20 to the body fat scale 10. The communication unit 110 corresponds to an example of a transmission request reception unit.

In addition, the communication unit 110 transmits, based on the above transmission request, the individual registration information for a plurality of persons stored in the individual information storage unit 141, to the communication unit 210. The communication unit 110 corresponds to an example of an individual registration information transmission unit.

The smartphone 20 displays the individual registration information transmitted by the communication unit 110, and thereby the user performing registration of individual registration information can confirm the registration situation of individual registration information in the individual information storage unit 141. Thus, the user performing registration of individual registration information can avoid the individual registration information being overwritten by mistake.

In addition, the communication unit 110 receives, from the communication unit 210, a registration request in which designation of any of the individual registration information for a plurality of persons stored in the individual information storage unit 141 and a registration content are associated. The communication unit 110 corresponds to an example of a registration request reception unit.

The communication system between the communication unit 110 and the communication unit 210 can employ a variety of methods. For example, the communication unit 110 and the communication unit 210 may perform wireless communication using radio waves, infrared light, or the like, or may perform wire communication.

The display unit 120, for example, has a display screen including a liquid-crystal-type digital panel meter and displays a variety of information such as measurement values of the body weight or body fat percentage in accordance with control of the display control unit 182.

The operation input unit 130, for example, has an input device such as a push-button-type setting button or a foot switch (switch operated by a foot) and accepts a user operation. Specifically, the operation input unit 130 accepts a user operation which commands pairing between the body fat scale 10 and the smartphone 20. The term "pairing" here denotes a process in which the apparatus to be a communication partner is registered. For example, the body fat scale 10 and the smartphone 20 detect each other to be in communication connection and each acquire and store the communication address of the partner.

The biological information sensor unit 150 has, for example, a sensor such as a load cell or an impedance sensor for measuring the body weight or biological impedance. The biological information sensor unit 150 performs measurement of the body weight or biological impedance in accordance with control of the biological information acquisition unit 185.

The control unit 180 controls each unit of the body fat scale 10 and implements a variety of functions. For example, a CPU (Central Processing Unit) provided in the body fat scale 10 reads out a program from the storage unit 140 and executes the program, and thereby the control unit 180 is realized. Alternatively, the control unit 180 may be realized in a configuration other than the configuration using a CPU. For example, the control unit 180 may be realized by hardware using a dedicated circuit.

The communication control unit 181 controls the communication unit 110 to perform communication with the communication unit 210. The display control unit 182 controls the display unit 120 to display a variety of information such as measurement values of the body weight or body fat percentage. The input process unit 183 detects the user operation accepted by the operation input unit 130.

The storage process unit 184 performs a variety of processes on the storage unit 140, such as writing to, reading from, or searching of data of the storage unit 140.

Specifically, the storage process unit 184 registers the registration content indicated in the registration request, on the individual registration information designated in the registration request, of the individual registration information stored in the individual information storage unit 141.

The storage process unit 184 corresponds to an example of a registration unit.

Further, the storage process unit 184 performs processing of data to be stored in the individual information storage unit 141. For example, the storage process unit 184 sets a control ID when the individual registration information is stored in the individual information storage unit 141.

The biological information acquisition unit 185 controls the biological information sensor unit 150 to acquire biological information. Specifically, the biological information acquisition unit 185 controls the biological information sensor unit 150 to perform measurement of the body weight and acquires a body weight measurement value. In addition, the biological information acquisition unit 185 controls the biological information sensor unit 150 to perform measurement of the biological impedance and calculates the body fat percentage based on the obtained biological impedance measurement value.

The communication process unit 186 performs a process in response to the transmission request of the individual registration information, which is received by the communication unit 110 from the communication unit 210. Specifically, when the communication unit 110 receives the transmission request of the individual registration information, the communication process unit 186 reads out the individual registration information stored in the individual information storage unit 141 via the storage process unit 184. Then, the communication process unit 186 transmits the individual registration information which is read out, via the communication unit 110 to the communication unit 210 which is the origin of the transmission request.

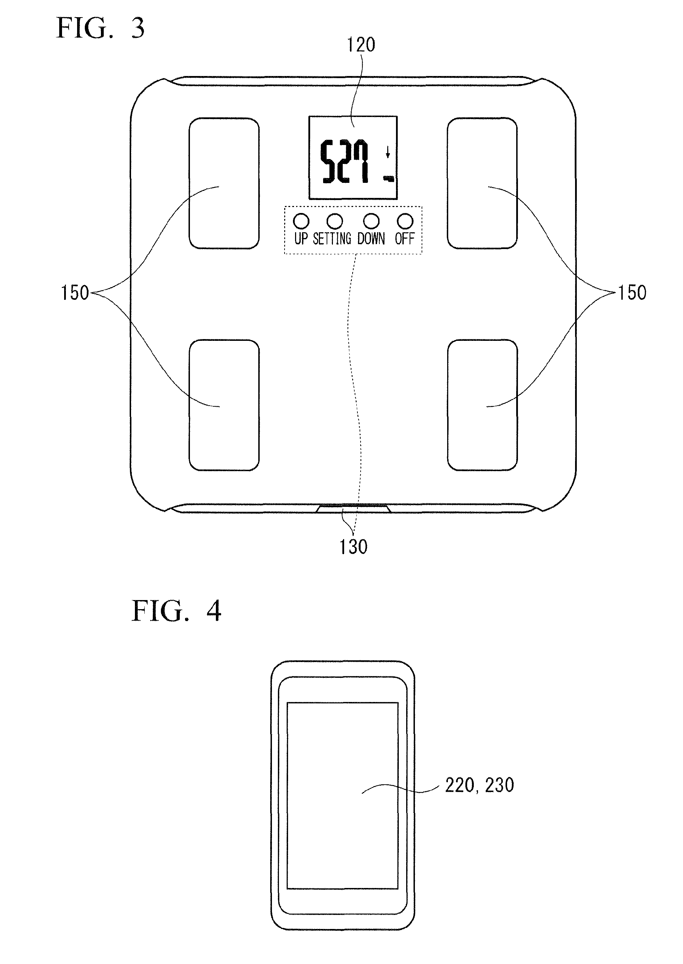

FIG. 3 is a schematic outline diagram showing an example of an external form of the body fat scale 10. In the drawing, the display screen of the display unit 120, the setting button and the foot switch of the operation input unit 130, and a measuring terminal (electrode) of the biological information sensor unit 150 are presented.

As described above, the display unit 120 displays a variety of information such as measurement values of the body weight or body fat percentage on the display screen.

The operation input unit 130 accepts a user operation of pressing the setting button or the foot switch. For example, the operation input unit 130 accepts a user operation which commands pairing between the body fat scale 10 and the smartphone 20 via an operation of pressing a predetermined setting button.

The biological information sensor unit 150 performs measurement of the body weight or biological impedance in accordance with control of the biological information acquisition unit 185 in a state where the user stands on the body fat scale 10 and the sole of the foot is in contact with the measuring terminal (electrode).

With reference back to FIG. 1, the smartphone 20 corresponds to an example of a terminal apparatus and functions as a terminal of the body fat scale 10. For example, the smartphone 20 performs a registration request of individual registration information to the body fat scale 10. Further, the smartphone 20 displays a measurement result of biological information by the body fat scale 10.

The communication unit 210 performs communication with the communication unit 110 of the body fat scale 10 in accordance with control of the communication control unit 281.

Specifically, the communication unit 210 transmits a transmission request of the individual registration information stored in the individual information storage unit 141 to the body fat scale 10, prior to a registration request of individual registration information. The communication unit 210 corresponds to an example of a transmission request transmission unit.

Then, the communication unit 210 receives the individual registration information for a plurality of persons stored in the individual information storage unit 141 from the communication unit 110 in response to the transmission request of the individual registration information. The communication unit 210 corresponds to an example of an individual registration information reception unit.

In addition, the communication unit 210 transmits, to the communication unit 210, a registration request in which designation of individual registration information selected in the selection operation of the user, of a plurality of individual registration information received in response to the transmission request of the individual registration information, and the registration content on the individual registration information are associated. The communication unit 210 corresponds to an example of a registration request transmission unit.

The display unit 220 has, for example, a display screen such as a liquid crystal panel or an organic EL (Organic Electro Luminescence) panel and displays a variety of information such as a measurement result of biological information in accordance with control of the display control unit 282. Specifically, the display unit 220 displays the individual registration information for a plurality of persons received by the communication unit 210. The display unit 220 corresponds to an example of an individual registration information notification unit.

However, the notification method of the smartphone 20 is not limited to a method by way of visual display. For example, the smartphone 20 may perform the notification of a variety of information by way of, for example, voice (audio).

The operation input unit 230 has a touch sensor which is provided on the display screen of the display unit 220 and constitutes a touch panel, and accepts a variety of user operations. Specifically, the operation input unit 230 accepts a selection operation of an object on which individual registration information is newly registered, of the individual registration information for a plurality of persons stored in the individual information storage unit 141.

The storage unit 240 is configured to include a storage device provided in the smartphone 20 and stores a variety of information in accordance with control of the storage process unit 284.

The control unit 280 controls each unit of the smartphone 20 and implements a variety of functions. For example, a CPU provided in the smartphone 20 reads out a program from the storage unit 240 and executes the program, and thereby the control unit 280 is realized.

The communication control unit 281 controls the communication unit 210 to perform communication with the communication unit 110. The display control unit 282 controls the display unit 220 to display a variety of information such as measurement values of the body weight or body fat percentage. The input process unit 283 detects the user operation accepted by the operation input unit 230. The storage process unit 284 performs a variety of processes on the storage unit 240, such as writing to, reading from, or searching of data of the storage unit 240.

The application implementation unit 285 executes a variety of applications and realizes a variety of functions. Specifically, the application implementation unit 285 executes an application for a terminal of the body fat scale 10, and thereby causes the smartphone 20 to function as a terminal apparatus of the body fat scale 10.

The registration presence or absence determination unit 286 determines whether or not the individual registration information selected by the user as the object on which individual registration information is newly registered of the individual registration information stored in the individual information storage unit 141 is in a registered state. The term "individual registration information is registered" here denotes that the individual registration information stored in the individual information storage unit 141 is in a registered state (not in an unregistered state but in a state where substantive data is stored therein).

For example, when the individual registration information of Individual 2 is selected as the object on which individual registration information is newly registered, the registration presence or absence determination unit 286 acquires the individual registration information of Individual 2 stored in the individual information storage unit 141, refers to the content of the individual registration information, and determines whether the individual registration information is in a registered state or in an unregistered state.

When the registration presence or absence determination unit 286 determines that the individual registration information designated in the registration request, of the individual registration information stored in the individual information storage unit 141 is in a registered state, the overwrite approval or rejection confirmation process unit 287 performs a confirmation process of overwrite approval or rejection of the individual registration information.

Specifically, the overwrite approval or rejection confirmation process unit 287 inquires overwrite approval or rejection (whether or not overwriting is approved) at the user via the display unit 220. Then, the overwrite approval or rejection confirmation process unit 287 acquires a reply by the user via the operation input unit 230. When the reply indicates overwrite rejection, the overwrite approval or rejection confirmation process unit 287 prevents transmission of the registration request of the individual registration information to the body fat scale 10. Thus, in the body fat scale 10, registration (writing) of the individual registration information on the individual information storage unit 141 by the storage process unit 184 is prevented.

FIG. 4 is a schematic outline diagram showing an example of an external form of the smartphone 20. In the drawing, a touch panel configured to include the display screen of the display unit 220 and the touch sensor of the operation input unit 230 is presented.

As described above, the display unit 220 displays a variety of information on the display screen. Then, the operation input unit 230 accepts a user operation by way of a touch operation onto the display screen of the display unit 220. Specifically, the display unit 220 displays the individual registration information stored in the individual information storage unit 141, and the operation input unit 230 accepts a selection operation of an object on which individual registration information is newly registered.

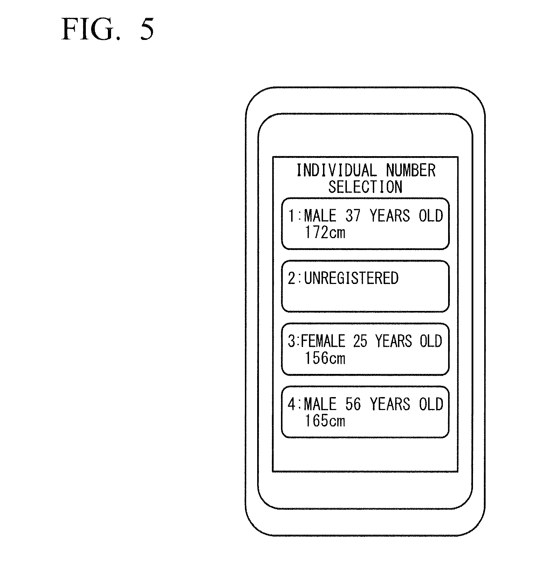

FIG. 5 is a diagram showing a display example of individual registration information stored in the individual information storage unit 141 by the display unit 220.

In the drawing, the display unit 220 displays the gender, age, and body height with respect to individual registration information for four persons of Individual 1 to Individual 4 stored in the individual information storage unit 141. Since the individual registration information of Individual 2 is in an unregistered state, the display unit 220 displays that the individual registration information is unregistered.

When the user performs a touch operation in which any of Individual 1 to Individual 4 is selected, the application implementation unit 285 generates a registration request which requests registration of new individual registration information onto the selected individual registration information and transmits the registration request via the communication unit 210 to the body fat scale 10.

In such a way, the display unit 220 displays the individual registration information stored in the individual information storage unit 141, and thereby the user performing registration of individual registration information can confirm the registration situation of individual registration information in the individual information storage unit 141. Thereby, the user performing registration of individual registration information can avoid a mistake of overwriting the individual registration information. For example, FIG. 5 shows that the individual registration information of Individual 2 is unregistered. By selecting Individual 2, the user can register new individual registration information to the body fat scale 10, while maintaining the registered individual registration information of Individual 1, Individual 3, and Individual 4.

The terminal apparatus of the body fat scale 10 is not limited to a smartphone. A variety of terminal apparatuses capable of communicating with the body fat scale 10 can be used. Further, the number of the terminal apparatus of the body fat scale 10 is not limited to one. For example, there may be a plurality of terminal apparatuses. For example, each of the users having individual registration information registered at the body fat scale 10 may have a smartphone and use the smartphone as the terminal apparatus of the body fat scale 10.

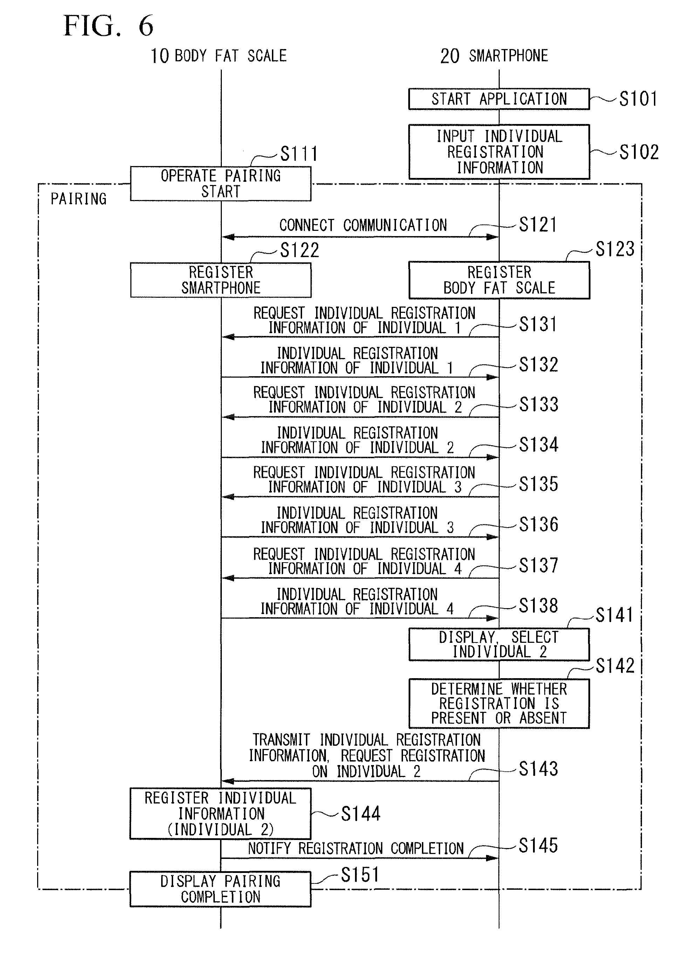

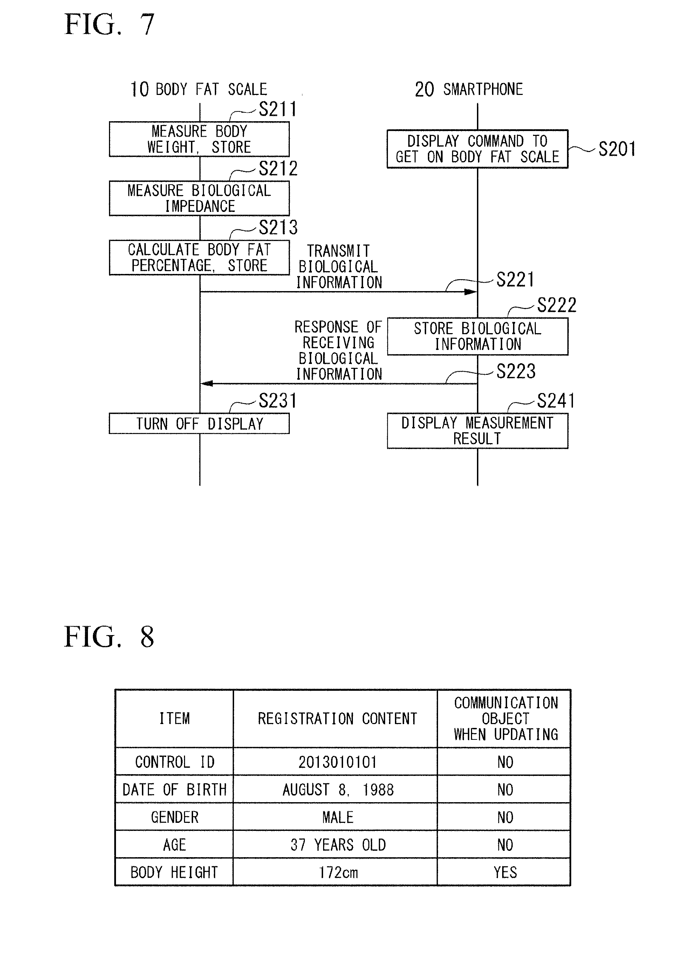

Next, with reference to FIG. 6 and FIG. 7, an operation of the biological information measuring system 1 is described.

FIG. 6 is a sequence diagram showing an example of a process sequence in which pairing between the body fat scale 10 and the smartphone 20 is performed.

In the process of the drawing, the application implementation unit 285 of the smartphone 20 starts an application for a terminal of the body fat scale 10 in accordance with a user operation (sequence S101).

Next, the operation input unit 230 accepts an input of individual registration information to be stored in the body fat scale 10, and the storage process unit 284 causes the storage unit 240 to store the input individual registration information (sequence S102).

On the other hand, in the body fat scale 10, the operation input unit 130 accepts a user operation which commands the start of pairing (sequence S111)). For example, the operation input unit 130 accepts the user operation which commands the start of pairing by way of a long press operation of the setting button shown in FIG. 3.

In response to the command of the pairing start, the body fat scale 10 and the smartphone 20 start pairing. Specifically, the body fat scale 10 and the smartphone 20 detect each other to be in communication connection (sequence S121). Then, the body fat scale 10 registers the smartphone 20 as a communication partner (sequence S122). On the other hand, the smartphone 20 registers the body fat scale 10 as a communication partner (sequence S123).

In addition, the body fat scale 10 and the smartphone 20 perform registration of individual registration information as a process associated with pairing. Specifically, first, the communication unit 210 requests transmission of the individual registration information of Individual 1 to Individual 4 stored in the individual information storage unit 141, to the communication unit 110, and the communication unit 110 replies the requested individual registration information (sequences S131 to S138).

Note that, when the number of the individual registration information capable of being registered in the body fat scale 10 is unknown to the smartphone 20, the communication unit 210 may once transmit, to the communication unit 110, a transmission request of all of the individual registration information.

In the smartphone 20 which acquires the individual registration information stored in the individual information storage unit 141, the display unit 220 displays the individual registration information, and the operation input unit 230 accepts a user operation of selecting any of the individual registration information (sequence S141). In the example of FIG. 6, the individual registration information of Individual 2 which is unregistered is selected.

Then, the registration presence or absence determination unit 286 determines whether registration of the individual registration information selected in the sequence S141 is present or absent (sequence S142). For example, the registration presence or absence determination unit 286 determines whether or not the content of the individual registration information obtained from the body fat scale 10 is data indicating absence of registration.

In the example of FIG. 6, since the individual registration information of Individual 2 is selected, the registration presence or absence determination unit 286 determines the content of the individual registration information of Individual 2 obtained in sequence S134. Since the individual registration information of Individual 2 is unregistered, the content of the individual registration information is data indicating absence of registration, and the registration presence or absence determination unit 286 determines that registration is absent.

When the registration presence or absence determination unit 286 determines that registration is present, the overwrite approval or rejection confirmation process unit 287 inquires overwrite approval or rejection (whether overwriting is approved or disapproved) at the user. For example, the overwrite approval or rejection confirmation process unit 287 causes the display unit 220 to display that the selected Individual 2 is registered and a message inquiring overwrite approval or rejection, and waits for a user operation of replying overwrite approval or rejection accepted by the operation input unit 230. When a reply of overwrite approval is obtained, the smartphone 20 subsequently performs a process of requesting registration to the body fat scale 10.

On the other hand, when a reply of overwrite rejection is obtained, the smartphone 20 does not transmit a registration request of the individual registration information to the body fat scale 10 and stops the process. Alternatively, the smartphone 20 (for example, overwrite approval or rejection confirmation process unit 287) may request the user to select other individual registration information.

When the registration presence or absence determination unit 286 determines that registration is absent, or when the overwrite approval or rejection confirmation process unit 287 detects a reply of overwrite approval, the communication unit 210 transmits a registration request which requests such that the individual registration information registered in sequence S102 is registered on Individual 2, to the communication unit 110 (sequence S143).

In the body fat scale 10, when the communication unit 110 receives a registration request from the smartphone 20, the storage process unit 184 registers the individual registration information on Individual 2 in accordance with the registration request (sequence S144).

Once the storage process unit 184 completes registration of the individual registration information in sequence S144, the communication unit 110 transmits a registration completion notification to the communication unit 210 (sequence S145).

The display unit 120 performs a display indicating that pairing has been completed (sequence S151).

After the process of FIG. 6, the body fat scale 10 and the smartphone 20 subsequently perform a process of FIG. 7.

FIG. 7 is a sequence diagram showing an example of a process sequence in which the body fat scale 10 measures biological information and the smartphone 20 displays a measurement result.

In the process of FIG. 7, the display unit 220 of the smartphone 20 displays a command that prompts the user to get on the body fat scale 10 (sequence S201).

In the body fat scale 10, the biological information sensor unit 150 performs a measurement of the body weight, and the storage process unit 184 causes the individual information storage unit 141 to store the measurement result (sequence S211). Further, the biological information sensor unit 150 performs a measurement of the biological impedance (sequence S212). The biological information acquisition unit 185 calculates the body fat percentage based on the biological impedance, and the storage process unit 184 causes the individual information storage unit 141 to store the calculation result (sequence S213).

Further, the communication unit 110 transmits the biological information (in the present embodiment, body weight and body fat percentage) to the communication unit 210 (sequence S221). In the smartphone 20, the communication unit 210 receives the biological information, and the storage process unit 284 causes the storage unit 240 to store the biological information (sequence S222).

Then, the communication unit 210 transmits a response notifying that the biological information has been received, to the communication unit 110 (sequence S223). The display unit 220 displays the biological information received by the communication unit 210. In this case, for example, a history of biological information including the received biological information may be displayed in a time series graph (sequence S241).

The body fat scale 10 which has received the response changes to a standby mode (a mode in which operation is stopped and power consumption is reduced), and the display unit 120 turns off the display (sequence S231).

Then, the process of FIG. 7 is completed.

As described above, the communication unit 110 of the body fat scale 10 transmits individual registration information for a plurality of persons stored in the individual information storage unit 141 to the smartphone 20 based on a request from the smartphone 20. The display unit 220 of the smartphone 20 displays the individual registration information, and thereby it is able to reduce a possibility that the user performing registration of individual registration information overwrites individual registration information by mistake. For example, the user can select unregistered individual registration information, and thereby register new individual registration information on the body fat scale 10 while maintaining registered individual registration information.

Further, the registration presence or absence determination unit 286 determines whether or not the individual registration information designated in the registration request is registered as the individual registration information stored in the individual information storage unit 141. Then, once the registration presence or absence determination unit 286 determines that registration is present, the overwrite approval or rejection confirmation process unit 287 inquires overwrite approval or rejection at the user.

In this way, the overwrite approval or rejection confirmation process unit 287 confirms overwrite approval or rejection, and thereby it is able to further reduce a possibility that the user performing registration of individual registration information overwrites individual registration information by mistake.

Note that, the storage process unit 184 updates the individual registration information stored in the individual information storage unit 141 in response to the request from the smartphone 20. For example, the body height of a growing child greatly changes. The storage process unit 184 updates the individual registration information stored in the individual information storage unit 141, and thereby the biological information acquisition unit 185 can calculate the body fat percentage further accurately.

When the update of the individual registration information stored in the individual information storage unit 141 is requested, the smartphone 20 may transmit only part of the individual registration information.

FIG. 8 is a diagram showing an example of a data structure of individual registration information stored in the storage unit 240, in an operation in which the smartphone 20 transmits only part of the individual registration information. In the example of FIG. 8, the storage unit 240 stores information for each item of the individual registration information indicating whether or not the item is a communication object at the time of update. The body height, which is one of the items shown in FIG. 8, is changeable and therefore is a communication object at the time of update. On the other hand, the date of birth or the gender is unchanged and therefore is not a communication object at the time of update. Further, the control ID is given by the body fat scale 10 and therefore is not a communication object at the time of update. Further, the age is calculated by the body fat scale 10 based on the date of birth and therefore is not a communication object at the time of update.

In such a way, by setting only part of the individual registration information to a communication object, the body fat scale 10 and the smartphone 20 can reduce the communication volume. Thus, it is possible to reduce the load of the communication process of the body fat scale 10 and the smartphone 20.

In addition to the body height described above, a setting of an athlete body type / a general body type, a menstrual cycle for a woman, and the like are examples of information which becomes a communication object when requesting update of the individual registration information stored in the individual information storage unit 141.

The storage process unit 184 may determine whether the update of the individual registration information is new or old.

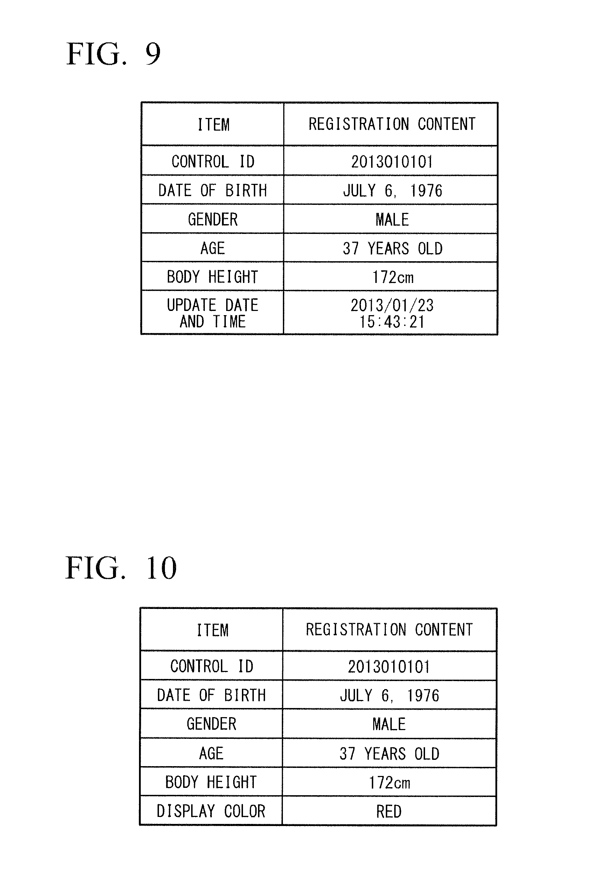

FIG. 9 is a diagram showing an example of a data structure of individual registration information stored in the individual information storage unit 141 and the storage unit 240, in an operation in which whether the update of the individual registration information is new or old is determined. In the example of FIG. 9, the individual information storage unit 141 and the storage unit 240 store individual registration information including a time stamp as update date and time information.

The communication unit 210 of the smartphone 20 transmits a registration request including update timing information in the individual registration information, and the communication unit 110 of the body fat scale 10 receives the registration request. Then, the storage process unit 184 compares the update timing information stored in the individual information storage unit 141 with the update timing information included in the registration request. The storage process unit 184 performs registration of individual registration information when the storage process unit 184 determines that an update timing (second update timing) indicated by the update timing information included in the registration request is newer than an update timing (first update timing) indicated by the update timing information stored in the individual information storage unit 141.

The storage process unit 184 may request the update of the information on the smartphone 20 (the transmission side of the registration request) when the storage process unit 184 determines that an update timing (first update timing) indicated by the update timing information stored in the individual information storage unit 141 is newer than an update timing (second update timing) indicated by the update timing information included in the registration request.

For example, the storage process unit 184 reads out appropriate information from the storage process unit 184, generates an update request, and transmits the update request to the smartphone 20 via the communication unit 110. In the smartphone 20, the communication unit 210 receives the update request. The storage process unit 284 reads out information from the update request and updates information stored in the storage unit 240.

In such a way, it is possible to update older information which is any one of information stored in the smartphone 20 and information stored in the body fat scale 10.

As described above, by determining whether the update of the individual registration information is new or old, the storage process unit 184 can perform the update further suitably even in a situation where a plurality of versions of the individual registration information are likely to arise. For example, in a situation where any of a plurality of smartphones 20 can perform a registration request of individual registration information, there can be a case in which a registration request based on newer data than that of a registration request received by the communication unit 110 has already been performed. In this case, the storage process unit 184 does not perform registration of individual registration information in response to the current registration request. Thus, the individual information storage unit 141 continues to store individual registration information based on newer data.

The body fat scale 10 and the smartphone 20 may perform a color classification display for each user.

FIG. 10 is a diagram showing an example of a data structure of individual registration information stored in the individual information storage unit 141 and the storage unit 240, in an operation in which color classification display of each user is performed. In the example of FIG. 10, the individual information storage unit 141 and the storage unit 240 store individual registration information including information of a display color of each user registered on Individual 1 to Individual 4. The color may be preliminarily determined, or may be selectable by the user at the time of registration of the individual registration information.

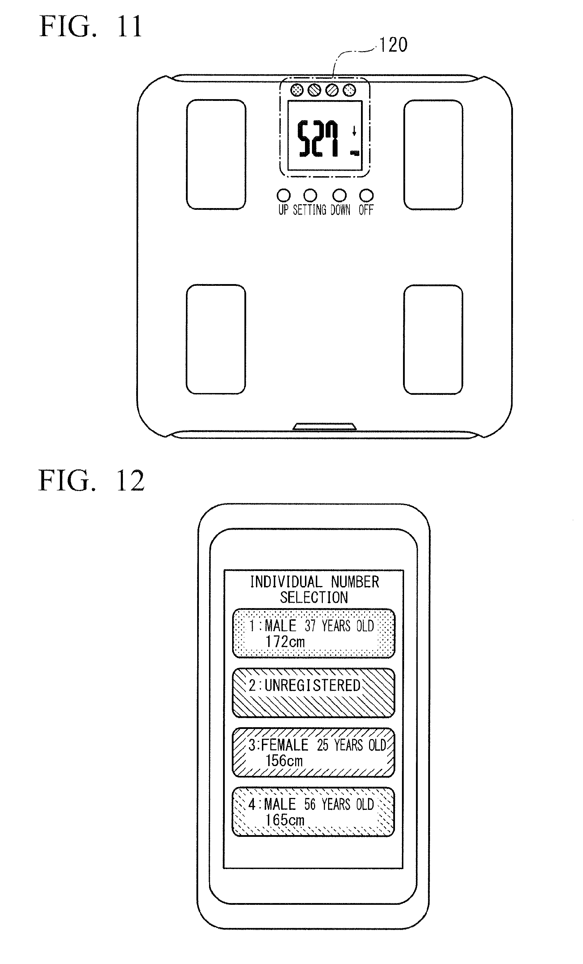

FIG. 11 is a schematic outline diagram showing an example of an external form of the body fat scale 10 in an operation in which color classification display of each user is performed. In the example of FIG. 11, the display unit 120 includes LEDs (Light Emitting Diodes) each corresponding to one of Individual 1 to Individual 4, in addition to the display screen described with reference to FIG. 3. The display unit 120 corresponds to an example of a color classification display unit. In FIG. 11, different patterns (hatching and dot) are used to illustrate that the display unit 120 includes LEDs each having a different color.

When individual information is measured, or when individual registration information is registered, the display unit 120 causes an LED having a color associated in the individual registration information with an object user to emit light.

The display unit 120 may perform color classification display in a display screen in addition to or alternatively to color classification display by LEDs. For example, the display unit 120 may cause the backlight of the display screen to emit light with a different color for each user. Alternatively, the display unit 120 may display a character having a different color for each user, on the display screen.

FIG. 12 is a diagram showing a display example of the smartphone 20 in an operation in which color classification display of each user is performed. In the example of FIG. 12, the display unit 220 of the smartphone 20 performs color classification and display of individual registration information stored in the individual information storage unit 141 using a color associated in the individual registration information. In FIG. 12, different patterns are used to illustrate that the display unit 120 performs display using a different color.

As described above, the display unit 120 performs display of a color when biological information is measured. The individual information storage unit 141 stores individual registration information including color information. The display unit 120 displays a color associated with a biological information measurement object person in the individual registration information.

In other words, the individual information storage unit 141 stores individual registration information including color information indicating a color associated with a biological information measurement object person. The display unit 120 displays the individual registration information using the color associated with the biological information measurement object person.

Accordingly, the user can recognize, by the color, the difference of the user on which individual registration information is registered and further easily differentiate the registered user and the unregistered user. Specifically, at the time of registration of individual registration information, the user can further easily understand the correspondence relation between the individual registration information and the user, and it is possible to reduce a possibility that information relating to a user which is already stored in the biological information measuring apparatus is overwritten by mistake.

The smartphone 20 can perform color classification display of the user, and thereby it is able to further reduce a possibility that information relating to a user which is already stored in the biological information measuring apparatus is overwritten by mistake.

An item (notification object item) to be a display object, of the items of individual registration information, may be selectable by the user.

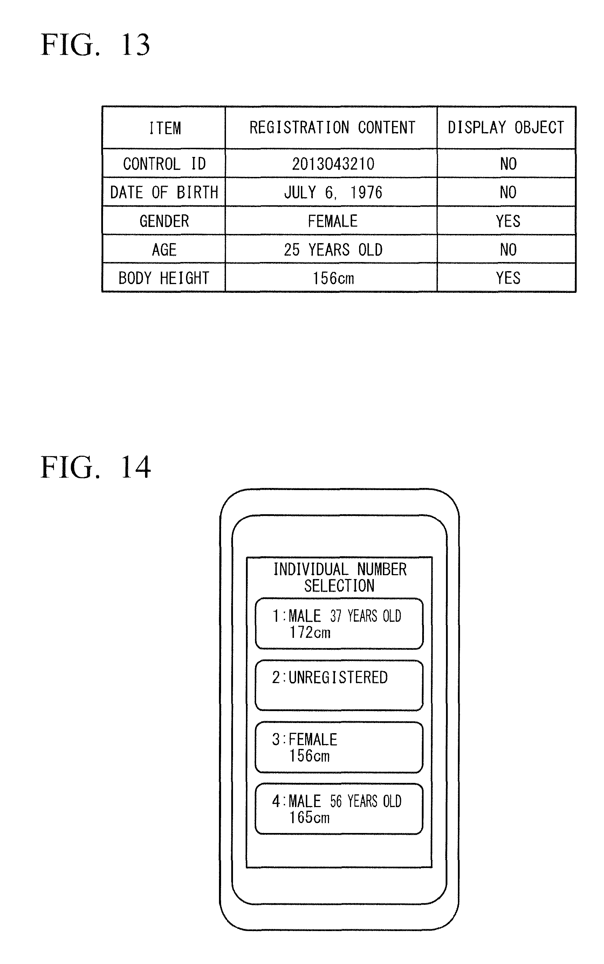

FIG. 13 is a diagram showing an example of a data structure of individual registration information stored in the individual information storage unit 141 and the storage unit 240, in an operation in which a user can select an item to be a display object, of the items of individual registration information. In the example of FIG. 13, the individual information storage unit 141 and the storage unit 240 store information indicating whether or not each item of the individual registration information is a display object.

For example, the communication unit 210 transmits a registration request including information indicating a display object item of the individual registration information, and the communication unit 110 receives the registration request. The display object item is set by the user performing a registration request operation.

The individual information storage unit 141 stores individual information including information indicating a display object item of the individual registration information. The communication unit 110 transmits individual registration information for a plurality of persons including information indicating a display object item of the individual registration information.

FIG. 14 is a diagram showing a display example of the smartphone 20 in an operation in which a user can select an item to be a display object, of the items of individual registration information. The display unit 220 of the smartphone 20 sets the gender and the body height to the display object and excludes the age from the display object, with respect to the individual registration information of Individual 3 in accordance with the individual registration information shown in FIG. 13.

In such a way, the smartphone 20 displays the item selected by the user, of the items of individual registration information, and thereby the user can set such that information which the user does not want to show other people is not displayed. In this regard, the biological information measuring system 1 can protect privacy.

Further, another operation is possible in which the smartphone 20 displays an item by which the user performing a registration request operation of individual registration information can easily recognize the user registered on the individual registration information.

Next, a modified example of the present embodiment is described. In the embodiment described above, the determination whether or not individual registration information is registered is performed by a smartphone. On the other hand, in the modified example, the determination is performed by a body fat scale.

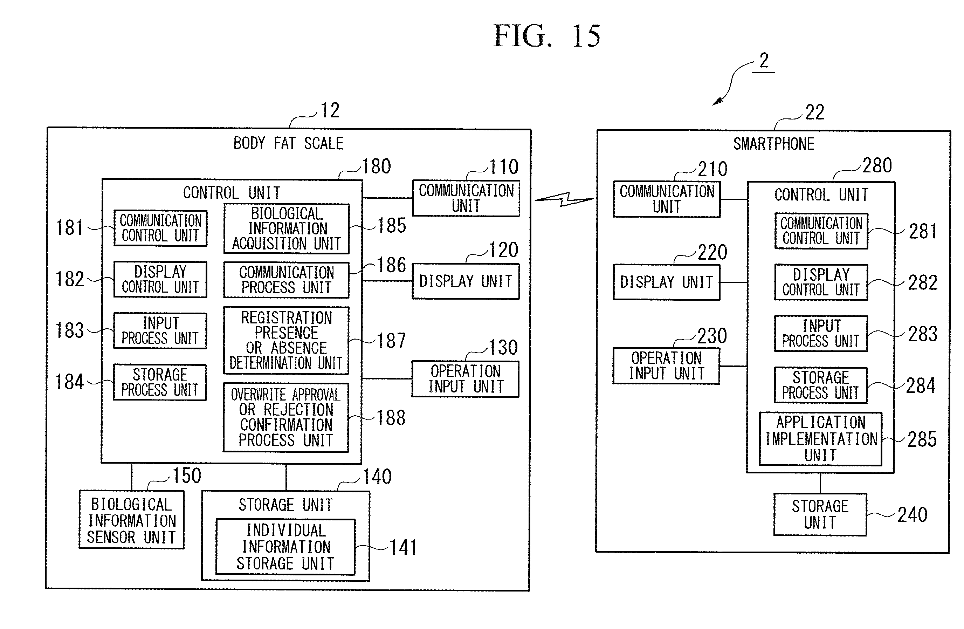

FIG. 15 is a schematic block diagram showing a functional configuration of a biological information measuring system according to a modified example of the present embodiment. In FIG. 15, a biological information measuring system 2 includes a body fat scale 12 and a smartphone 22.

The body fat scale 12 is provided with a communication unit 110, a display unit 120, an operation input unit 130, a storage unit 140, a biological information sensor unit 150, and a control unit 180. The storage unit 140 is provided with an individual information storage unit 141. The control unit 180 is provided with a communication control unit 181, a display control unit 182, an input process unit 183, a storage process unit 184, a biological information acquisition unit 185, a communication process unit 186, a registration presence or absence determination unit 187, and an overwrite approval or rejection confirmation process unit 188. The smartphone 22 is provided with a communication unit 210, a display unit 220, an operation input unit 230, a storage unit 240, and a control unit 280. The control unit 280 is provided with a communication control unit 281, a display control unit 282, an input process unit 283, a storage process unit 284, and an application implementation unit 285.

In FIG. 15, the units having a similar function corresponding to each unit of FIG. 1 are given the same descriptive symbols (110, 120, 130, 140, 141, 150, 180 to 186, 210, 220, 230, 240, and 280 to 285).

The body fat scale 12 measures the body weight and body fat percentage of a user and stores a history of measurement results, similarly as the body fat scale 10 (FIG. 1).

The body fat scale 12 is capable of registering a plurality of users. The body fat scale 12 stores a history of measurement results for each user. Further, the body fat scale 12 performs communication with the smartphone 22 and exchanges a variety of information relating to the user of the body fat scale 12, such as a measurement result of biological information.

The body fat scale 12 corresponds to an example of a biological information measuring apparatus. The biological information measuring apparatus of the present modified example is not limited to a body fat scale, and can be various kinds of biological information measuring apparatuses such as a blood pressure monitor or an electrocardiogram monitor.

The storage unit 140 and the individual information storage unit 141 are similar as those in FIG. 1, and the description thereof is omitted.

The communication unit 110 performs communication with the communication unit 210 of the smartphone 22 in accordance with control of the communication control unit 181 and exchanges a variety of information, similarly as the case in FIG. 1. The communication unit 110 is configured to include a communication circuit provided in the body fat scale 12.

Specifically, the communication unit 110 receives, from the communication unit 210 of the smartphone 22, a transmission request of individual registration information and transmits the individual registration information to the communication unit 210, and the communication unit 110 receives a registration request of individual registration information from the communication unit 210, similarly as the case in FIG. 1.

Additionally, when the registration presence or absence determination unit 187 determines that the individual registration information designated in the registration request has been registered, the communication unit 110 transmits a confirmation request of overwrite approval or rejection to the communication unit 210.

The communication unit 110 corresponds to an example of an overwrite approval or rejection confirmation request transmission unit.

Then, the communication unit 110 receives a reply to the confirmation request of overwrite approval or rejection. The communication unit 110 corresponds to an example of an overwrite approval or rejection reply reception unit.

In this way, the body fat scale 12 requests confirmation of overwrite approval or rejection, and thereby it is able to further reduce a possibility that the user performing registration of individual registration information overwrites individual registration information by mistake.

The communication system between the communication unit 110 and the communication unit 210 can employ a variety of methods similarly as the case in FIG. 1.

The display unit 120, the operation input unit 130, and the biological information sensor unit 150 are similar as those in FIG. 1, and the description of each unit is omitted.

The control unit 180 controls each unit of the body fat scale 12 and implements a variety of functions, similarly as the case in FIG. 1. For example, a CPU (Central Processing Unit) provided in the body fat scale 12 reads out a program from the storage unit 140 and executes the program, and thereby the control unit 180 is realized. Alternatively, the control unit 180 may be realized in a configuration other than the configuration using a CPU. For example, the control unit 180 may be realized by hardware using a dedicated circuit.

The communication control unit 181, the display control unit 182, the input process unit 183, the biological information acquisition unit 185, and the communication process unit 186 are similar as those in FIG. 1, and the description thereof is omitted.

The storage process unit 184 performs a variety of processes to the storage unit 140, such as writing, reading, or searching of data to the storage unit 140.

Specifically, the storage process unit 184 registers the registration content indicated in the registration request, on the individual registration information designated in the registration request, of the individual registration information stored in the individual information storage unit 141.

When the communication unit 110 receives a reply to the overwrite approval or rejection confirmation request of the individual registration information, the storage process unit 184 prevents registration of information indicated in the reply as overwrite rejection (specifically, does not perform registration). The storage process unit 184 corresponds to an example of a registration unit.

Further, the storage process unit 184 performs processing of data to be stored in the individual information storage unit 141. For example, the storage process unit 184 sets a control ID when the individual registration information is stored in the individual information storage unit 141.

The registration presence or absence determination unit 187 determines whether or not the individual registration information designated in the registration request received from the communication unit 210 by the communication unit 110, of the individual registration information stored in the individual information storage unit 141, is in a registered state. For example, when the communication unit 110 receives the registration request of the individual registration information on Individual 2, the registration presence or absence determination unit 187 reads out the individual registration information of Individual 2 stored in the individual information storage unit 141 via the storage process unit 184. Then, the registration presence or absence determination unit 187 refers to the content of the individual registration information which is read out, and determines whether the individual registration information of Individual 2 is in a registered state or in an unregistered state.

When the registration presence or absence determination unit 187 determines that the individual registration information designated in the registration request, of the individual registration information stored in the individual information storage unit 141, is in a registered state, the overwrite approval or rejection confirmation process unit 188 performs a confirmation process of overwrite approval or rejection of the individual registration information.

Specifically, the overwrite approval or rejection confirmation process unit 188 transmits an inquiry of overwrite approval or rejection to the communication unit 210 via the communication unit 110. Then, the overwrite approval or rejection confirmation process unit 188 acquires a reply transmitted from the communication unit 210 via the communication unit 110. When the reply indicates overwrite rejection, the overwrite approval or rejection confirmation process unit 188 does not perform registration (writing) of the individual registration information on the individual information storage unit 141 by the storage process unit 184.

The smartphone 22 corresponds to an example of a terminal apparatus and functions as a terminal of the body fat scale 12, similarly as the case in FIG. 1. For example, the smartphone 22 performs a registration request of individual registration information to the body fat scale 12. Further, the smartphone 22 displays a measurement result of biological information by the body fat scale 12.

The communication unit 210 performs communication with the communication unit 110 of the body fat scale 12 in accordance with control of the communication control unit 281, similarly as the case in FIG. 1.

Specifically, the communication unit 210 transmits a transmission request of individual registration information to the body fat scale 12, receives the individual registration information from the communication unit 110, and transmits a registration request of individual registration information to the communication unit 110, similarly as the case in FIG. 1.

Additionally, the communication unit 210 receives a confirmation request of overwrite approval or rejection of individual registration information from the communication unit 110. Then, the communication unit 210 transmits a reply to the confirmation request of overwrite approval or rejection to the communication unit 110.

The display unit 220, the operation input unit 230, and the storage unit 240 are similar as those in FIG. 1, and the description thereof is omitted.

The control unit 280 controls each unit of the smartphone 22 and implements a variety of functions, similarly as the case in FIG. 1. For example, a CPU provided in the smartphone 22 reads out a program from the storage unit 240 and executes the program, and thereby the control unit 280 is realized.

The communication control unit 281, the display control unit 282, the input process unit 283, the storage process unit 284, and the application implementation unit 285 are similar as those in FIG. 1, and the description thereof is omitted.

The terminal apparatus of the body fat scale 12 is not limited to a smartphone, similarly as the case in FIG. 1. Further, the number of the terminal apparatus of the body fat scale 12 is not limited to one.

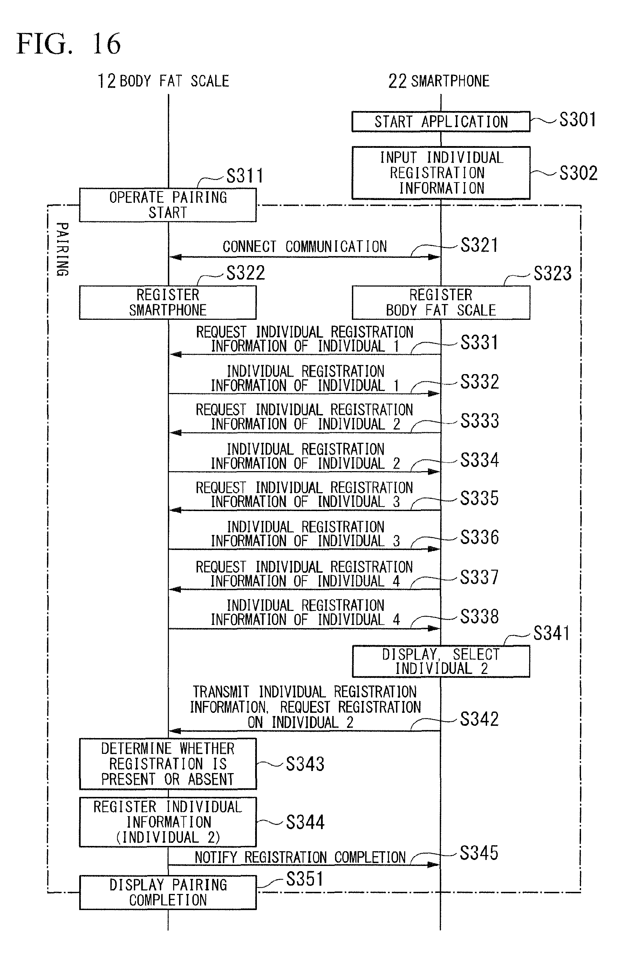

Next, with reference to FIG. 16, an operation of the biological information measuring system 2 is described.

FIG. 16 is a sequence diagram showing an example of a process sequence in which pairing between the body fat scale 12 and the smartphone 22 is performed. Sequences S301 to S341 are similar as sequences S101 to S141 in FIG. 6.

After sequence S341, the communication unit 210 transmits a registration request which requests such that the individual registration information registered in sequence S302 is registered on Individual 2, to the communication unit 110 (sequence S342).

In the body fat scale 12, the registration presence or absence determination unit 187 determines whether registration of the individual registration information selected in the registration request is present or absent (sequence S343). For example, the registration presence or absence determination unit 187 reads out the individual registration information of Individual 2 selected in the registration request from the individual information storage unit 141 via the storage process unit 184. Then, the registration presence or absence determination unit 187 determines whether or not the content of the individual registration information which is read out is data indicating absence of registration.

Since unregistered individual registration information is selected in sequence S341, the registration presence or absence determination unit 187 determines that registration is absent. Then, the storage process unit 184 registers the individual registration information on Individual 2 in accordance with the registration request (sequence S344).

When the registration presence or absence determination unit 187 determines that registration is present, the overwrite approval or rejection confirmation process unit 188 inquires overwrite approval or rejection at the smartphone 22 via the communication unit 110. When a reply of overwrite approval is obtained, the storage process unit 184 registers the individual registration information on Individual 2 in accordance with a registration request. On the other hand, when a reply of overwrite rejection is obtained, the storage process unit 184 does not perform registration of the individual registration information.

Sequences S345 to S351 are similar as sequences S145 to S151 in FIG. 6. The process after FIG. 16 is also similar as the process after FIG. 6 described above.

As described above, the communication unit 110 of the body fat scale 12 transmits individual registration information for a plurality of persons stored in the individual information storage unit 141 to the smartphone 22 based on a request from the smartphone 22, similarly as the case in FIG. 1. The display unit 220 of the smartphone 22 displays the individual registration information, and thereby it is able to reduce a possibility that the user performing registration of individual registration information overwrites individual registration information by mistake. For example, the user can select unregistered individual registration information, and thereby register new individual registration information on the body fat scale 12 while maintaining registered individual registration information.

Further, the registration presence or absence determination unit 187 determines whether or not the individual registration information designated in the registration request is registered as the individual registration information stored in the individual information storage unit 141. Then, once the registration presence or absence determination unit 187 determines that registration is present, the overwrite approval or rejection confirmation process unit 188 transmits a confirmation request of overwrite approval or rejection to the smartphone 22.

In this way, the body fat scale 12 requests confirmation of overwrite approval or rejection, and thereby it is able to further reduce a possibility that the user performing registration of individual registration information overwrites individual registration information by mistake.

Update of individual registration information, color classification of display, and user selection of a display object item are similar as those of the biological information measuring system 1 described above.

A program for realizing the functions of the control unit 180 or the control unit 280 in whole or in part may be recorded on a computer-readable recording medium. In this case, a computer system may read the program recorded on the recording medium and may execute the program, to thereby perform the process of each unit. Here, the "computer system" includes an OS and hardware such as a peripheral device.

Further, when the WWW system is used, the "computer system" includes a homepage providing environment (or a display environment).

Examples of the "computer-readable recording medium" include portable media, such as a flexible disk, a magneto-optical disc, a ROM, and a CD-ROM, and a storage device, such as a hard disk built into a computer system. Moreover, examples of the "computer-readable recording medium" include a recording medium that stores a program dynamically for a short period of time like a network, such as the Internet, or a communication line when a program is transmitted through a communication line, such as a telephone line, and may include a recording medium that stores a program for a predetermined period of time like a volatile memory in a computer system which serves as a server or a client in this case. Further, the above program may be a program for realizing some of the functions described above or may be a program capable of realizing the above functions by combination with a program already recorded in the computer system.

While the embodiments of the present invention have been described above in detail with reference to the accompanying drawings, it should be noted that the specific configuration is not limited to the embodiments, and various design modifications thereof can be made without departing from the scope of the present invention.

## Claims

1. A biological information measuring apparatus comprising:
an individual information storage unit configured to store individual information for a plurality of persons in which individual registration information and biological information are associated;
a transmission request reception unit configured to receive a transmission request of the individual registration information;
an individual registration information transmission unit configured to transmit the individual registration information for a plurality of persons stored in the individual information storage unit based on the transmission request;
a registration request reception unit configured to receive a registration request in which designation of any of the individual registration information for a plurality of persons and a registration content are associated; and
a registration unit configured to register the registration content on the individual registration information designated in the registration request of the individual registration information stored in the individual information storage unit.

2. The biological information measuring apparatus according to claim 1, wherein
the individual information storage unit stores the individual registration information including update timing information,
the registration request reception unit receives the registration request including update timing information in the individual registration information, and
the registration unit compares the update timing information stored in the individual information storage unit with the update timing information included in the registration request and performs registration of individual registration information when the registration unit determines that an update timing indicated by the update timing information included in the registration request is newer.

3. The biological information measuring apparatus according to claim 2, wherein
the registration unit requests, to a transmission side of the registration request, the update of the individual registration information when the registration unit determines that an update timing indicated by the update timing information stored in the individual information storage unit is newer than an update timing indicated by the update timing information included in the registration request.

4. The biological information measuring apparatus according to any one of claims 1 to 3, comprising:
a registration presence or absence determination unit configured to determine whether or not the individual registration information designated in the registration request is registered as the individual registration information stored in the individual information storage unit;
an overwrite approval or rejection confirmation request transmission unit configured to transmit a confirmation request of overwrite approval or rejection when the registration presence or absence determination unit determines that registration is present; and
an overwrite approval or rejection reply reception unit configured to receive a reply to the confirmation request of overwrite approval or rejection, wherein
the registration unit prevents registration of information indicated in the reply as overwrite rejection.

5. The biological information measuring apparatus according to any one of claims 1 to 4, comprising:
a color classification display unit configured to perform display of a color when biological information is measured, wherein
the individual information storage unit stores the individual registration information including color information, and
the color classification display unit displays a color associated with a biological information measurement object person in the individual registration information.

6. The biological information measuring apparatus according to any one of claims 1 to 5, wherein
the registration request reception unit receives the registration request including information indicating a notification object item of the individual registration information,
the individual information storage unit stores the individual information including information indicating a notification object item of the individual registration information, and
the individual registration information transmission unit transmits the individual registration information for a plurality of persons including information indicating a notification object item of the individual registration information.

7. A biological information measuring system comprising:
a biological information measuring apparatus; and a terminal apparatus, wherein
the biological information measuring apparatus comprises:
an individual information storage unit configured to store individual information for a plurality of persons in which individual registration information and biological information are associated;
a transmission request reception unit configured to receive a transmission request of the individual registration information from the terminal apparatus;
an individual registration information transmission unit configured to transmit the individual registration information for a plurality of persons stored in the individual information storage unit to the terminal apparatus based on the transmission request;
a registration request reception unit configured to receive a registration request in which designation of any of the individual registration information for a plurality of persons and a registration content are associated, from the terminal apparatus; and
a registration unit configured to register the registration content on the individual registration information designated in the registration request of the individual registration information stored in the individual information storage unit, and
the terminal apparatus comprises:
a transmission request transmission unit configured to transmit a transmission request of the individual registration information to the biological information measuring apparatus;
an individual registration information reception unit configured to receive the individual registration information for a plurality of persons from the biological information measuring apparatus;
an individual registration information notification unit configured to notify the individual registration information for a plurality of persons received by the individual registration information reception unit;
an operation input unit configured to accept a selection operation of any of the individual registration information for a plurality of persons; and
a registration request transmission unit configured to transmit a registration request in which designation of the individual registration information selected in the selection operation and a registration content on the individual registration information are associated, to the biological information measuring apparatus.

8. An individual registration information registering method of a biological information measuring apparatus comprising an individual information storage unit configured to store individual information for a plurality of persons in which individual registration information and biological information are associated, the method comprising:
a transmission request reception step of receiving a transmission request of the individual registration information;
an individual registration information transmission step of transmitting the individual registration information for a plurality of persons stored in the individual information storage unit based on the transmission request;
a registration request reception step of receiving a registration request in which designation of any of the individual registration information for a plurality of persons and a registration content are associated; and
a registration step of registering the registration content on the individual registration information designated in the registration request of the individual registration information stored in the individual information storage unit.

9. A program for causing a terminal apparatus configured to perform communication with a biological information measuring apparatus to execute:
a transmission request transmission step of transmitting a transmission request of individual registration information to the biological information measuring apparatus;
an individual registration information reception step of receiving the individual registration information for a plurality of persons from the biological information measuring apparatus;
an individual registration information notification step of notifying the individual registration information for a plurality of persons received in the individual registration information reception step;
an operation input step of accepting a selection operation of any of the individual registration information for a plurality of persons; and
a registration request transmission step of transmitting a registration request in which designation of the individual registration information selected in the selection operation and a registration content on the individual registration information are associated, to the biological information measuring apparatus.
